Europäisches Patentamt

European Patent Office

Office européen des brevets

(11) Publication number: **0 517 916 A1**

# EUROPEAN PATENT APPLICATION
## published in accordance with Art. 158(3) EPC

(21) Application number: **91905334.8**

(22) Date of filing: **28.02.91**

(86) International application number:
**PCT/JP91/00267**

(87) International publication number:
**WO 91/12810 (05.09.91 91/21)**

(51) Int. Cl.5: **A61K 31/70**

(30) Priority: **01.03.90 JP 50320/90**

(43) Date of publication of application:
**16.12.92 Bulletin 92/51**

(84) Designated Contracting States:
**DE FR GB**

(71) Applicant: **LIMITED IMAGE MAKER**
**13-4, Nishikori 1-chome**
**Otsu-shi, Shiga 520(JP)**
Applicant: **HACHIDA, Mitsuhiro**
**Raionzu Puraza Kitaayase, 115, 3-12-23,**
**Otanida**
**Adachi-ku, Tokyo 120(JP)**

(72) Inventor: **HACHIDA, Mitsuhiro**
**Raionzu Puraza Kitaayase, 115, 3-12-23,**
**Otanida**
**Adachi-ku, Tokyo 120(JP)**

(74) Representative: **Ford, Michael Frederick et al**
**MEWBURN ELLIS & CO. 2/3 Cursitor Street**
**London EC4A 1BO(GB)**

(54) **IMMUNOSUPPRESSANT.**

(57) N-Acetylneuraminosylgalactosylceramide which is one of the complex glycosphingolipids mainly present in an outer membrane of a cell is quite effective in preventing immunorejection in organ transplantation.

EP 0 517 916 A1

TECHNICAL FIELD

The present invention relates to an immunosuppressive agent, in particular, an immunosuppressive agent capable of preventing rejection in organ transplantation.

BACKGROUND ART

Organs such as liver, heart and kidney individually have a function necessary for maintenance of life. At the time when the function of such an organ is impaired to such an extent that the organ cannot maintain the life, namely, at the last Stage of hepatocirrhosis, myocardosis, chronic nephritis, etc., survival can be made possible only by either making up for the deficiency of function of the organ by the use of an artificial organ, or replacing the organ having the impaired function by carrying out organ transplantation. However, artificial organs other than artificial kidney have not yet been developed to such a degree that they can maintain the life for a long period of time. Artificial kidney is a large machine installed in a hospital and does not enable a patient to live socially with this machine in his body. As to its function, it is effective in removing waste products but cannot be assisted by a function of internally secreting erythropoietic stimulating factors such as erythropoietin, which natural kidney has.

Therefore, organ transplantation is a practical and effective remedy for a patient who is in peril of life because of hypofunction of an organ. The organ transplantation, however, requires donation of the organ from another person. After the transplantation, there is a problem of rejection which is an immunoreaction with the antigen of the organ of the another person (which is called a transplantation antigen or a histocompatibility antigen and includes H-2 antigen of mice and HLA antigen of human beings).

As measures to counter the rejection, two measures are now clinically carried out. One of them is to conduct organ transplantation by choosing an organ donor and a patient who receives the organ transplant (a recipient), so that they may have the same histocompatibility antigen (HLA). The other is to suppress the rejection by administering an immunosuppressive agent after transplantation.

For the latter, i.e., the suppression of the rejection by the administration of an immunosuppressive agent, immunosuppressive agents such as cyclosporine have heretofore been used.

The conventional immunosuppressive agents such as cyclosporine, however, are disadvantageous in that they involve a great risk of causing organ toxicity or infections.

DISCLOSURE OF THE INVENTION

An object of the present invention is to provide an immunosuppressive agent comprising N-acetylneuraminyl-galactosylceramide as an active ingredient. The immunosuppressive agent of the present invention solves the problems in the conventional immunosuppressive agents and is effective particularly in preventing immunological rejection in organ transplantation.

Another object of the present invention is to provide an immunosuppression method comprising administration of an effective amount of N-acetylneuraminyl-galastosylceramide to any of warm-blooded animals including human beings.

Still another object of the present invention is to provide use of N-acetylneuraminylgalactosylceramide for production of an immunosuppressive agent.

BRIEF DESCRIPTION OF THE DRAWINGS

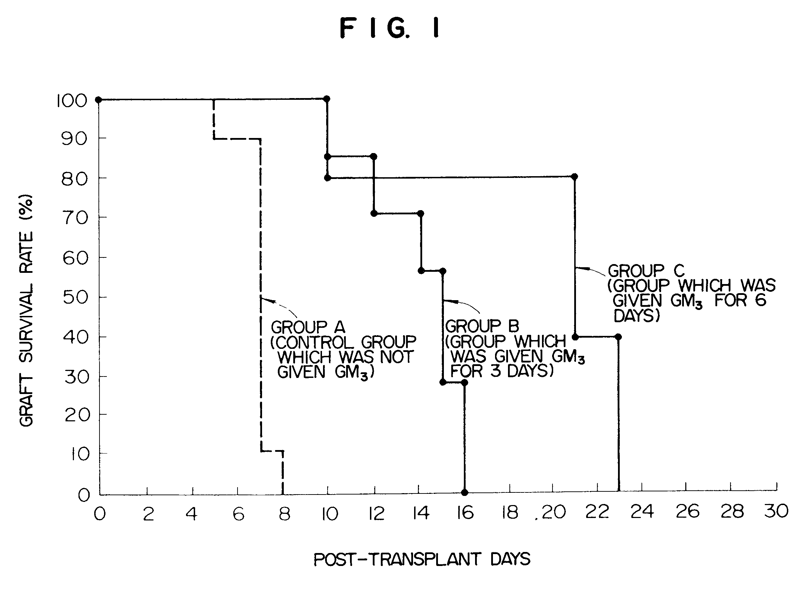

Fig. 1 is a graph in which the change with time of the graft survival time of a group to which the immunosuppressive agent of the present invention was administered for 3 days (group B) and a group to which said agent was administered for 6 days (group C) is compared with that of a control group to which said agent was not administered (group A).

BEST MODE FOR CARRYING OUT THE INVENTION

N-acetylneuraminyl-galactosylceramide (hereinafter abbreviated as $GM_3$) which is an active ingredient in the immunosuppressive agent of the present invention has a structure represented by the formula:

$$\text{Gal--Glc--cer} \\ | \\ \text{NeuAc}$$

wherein Gal is galactose, Glc is glucose, NeuAc is N-acetylneuraminate, and cer is a ceramide, and it is a complex glycosphingolipid found primarily on the external membrane of cells. $GM_3$ has been isolated from brain, spleen and erythrocyte. Bovine $GM_3$ is commercially available from Sigma Chemical Co. (St. Louis, USA). According to Glyco-conjugate J. 2, 5-9 (1985), $GM_3$ has been obtained by a synthetic method.

The immunosuppressive agent of the present invention comprising $GM_3$ as active ingredient is effective particularly in preventing immunological rejection in organ transplantation.

As organ transplantations in which said immunosuppressive agent can be used, there may be exemplified heart transplantation, lung transplantation, kidney transplantation, kidney transplantation, pancreas transplantation, liver transplantation, intestine transplantation, xenogenic transplantations, vein hetero-transplantation, etc. The immunosuppressive agent of the present invention comprising $GM_3$ as active ingredient is effective also in curing autoimmune diseases.

$GM_3$ can easily be absorbed by cells or tissues and hence can be administered directly into a graft (or an organ) by the use of a catheter or the like. Therefore, local immunosuppression can be carried out using $GM_3$, so that systemic side effects observed in the case of conventional immunosuppressive agents such as cyclosporine can be reduced.

According to the results of experiments using rats, the dose of $GM_3$ is preferably about 2 mg or more per 100 g (based on wet basis) of an organ.

The immunosuppressive agent of the present invention can be prepared using $GM_3$ and a conventional pharmacologically acceptable carrier. The immunosuppressive agent can be prepared, for example, by dissolving $GM_3$ in physiological saline immediately before use. The immunosuppressive agent can be prepared also by adding a carrier such as albumin to $GM_3$ and freeze-drying the resulting mixture.

$GM_3$ is a substance present in a living body, and hence it was confirmed that $GM_3$ has neither systemic nor local toxicity.

Investigation by the present inventor suggests the following two possible mechanisms (a) and (b) as mechanisms of the immunosuppressive effect of $GM_3$:

(a) $GM_3$ absorbed by a tissue changes the antigenecity of a graft to make difficult the invasion of the tissue by antibody.
(b) Free $GM_3$ interacts with lymphocytes, macrophages or other immunologically involved cells to exhibit its immunosuppressive effect.

EXAMPLES

The present invention is further explained below with reference to examples.

Inbred male rats (body wt. 250 to 300 g) were used in all experiments. Lewis rats (Lew, RT[1]) acted as organ recipients, and Buffalo rats (Buf, RT1[b]) were used as heart and lung donors.

The immunosuppressive agent of the present invention was prepared by dissolving $GM_3$ purified from bovine brain (which was available from Sigma Chemical Co., St. Louis, USA) in a saline solution prior to use.

Twenty-nine heterotopic heart and lung transplantations were carried out in three groups. Nine rats in group A were not given $GM_3$ and served as a control. Twelve rats in group B were given 300 $\mu$g of $GM_3$ daily for 3 consecutive days after transplantation. Eight rats in group C were given 300 $\mu$g of $GM_3$ daily for 6 consecutive days after transplantation. The transplantation operation was carried out according to the method of Lee et al. [Lee et al. Am. J. Pathol, 59, 279-298 (1970) and Transplant 33, 438-442 (1982)]. In detail, the donor rats were prepared in a routine fashion under anesthesia with ether and nembutal, using sterile techniques. The heart and lungs were removed from the donor after 0.3 ml of heparin was injected. The silastic tube (Silastic Medical-Grade Tubing, 0.02 in., mfd. by Dow-Corning Co., MI, USA) was placed into the inferior vena cava and secured with 4-O silk. Thus, the immunosuppressive agent comprising $GM_3$ was administered directly into the graft through the inferior vena cava.

In the recipient rats, the abdominal vein and artery were exposed and clamped using a noncrushing vascular clamp. An appropriate opening on the abdominal vena cava was made, and the graft's artery was anastomosed in an end-to-side fashion employing 8-O prolene suture (mfd. by Ethicon Inc, NJ, USA). Pulsation of the transplanted heart commenced spontaneously as the heart and the lungs regained circulation following removal of the clamp. The mean ischemic time of the allograft was approximately 17 minutes. The end of the catheter was introduced to the outside of abdomen. Through the silastic tube, 300 $\mu$g of $GM_3$ was administered daily for up to 3 days in group B and 6 days in group C.

The effect of $GM_3$ was evaluated by two different indications, i.e., graft survival time and histological findings of the graft. The cardiac activity was assessed every day by palpation through the flank. Whether the transplanted organs had been rejected or not was judged by employing as an indication a point of time at which myocardial contractions ceased completely.

For evaluation of the histological change of the allograft rejection, five rats in group B were sacrificed on day 7 and three rats in group C on day 14. The excised allografts were immediately fixed in 10% formal in and embedded in paraffin. Sections of 4 $\mu$ in thickness were cut out and treated by a conventional method and hence with hematoxyl in and eosin stain. It was carried out according to the Billingham's classification described in Histological Assessment, Heart and Vessels 1 (Suppl), 86-90 (1985).

Fig. 1 illustrates the graft survival time in each group. As can be seen from Fig. 1, the mean graft survival time was 6.9 ± 0.78 days (6 to 8 days) in group A, i.e., the control group, 14 ± 2.2 days (10 to 16 days) in group B, i.e., the $GM_3$-treated group, and 21.0 ±2.5 days (10 to 23 days) in group C, i.e., the another $GM_3$-treated group. A significant difference of survival times was observed between groups A and B, A and C, and B and C. (p < 0.01). The histological findings were compared between groups A and B on day 7, upon which eight out of the nine rats in group A had died but none of the 12 rats in group B had died. In group A, the myocyte degeneration became prominent and infiltration with numerous lymphocytes was observed. The interstitial lymphocyte infiltration and myocyte necrosis were found throughout the myocardial tissue in both ventricles.

On the other hand, the grafts in group B showed little lymphocyte infiltration at perivascular and interstitial areas of the myocardial tissue. Neither interstitial hemorrhage nor myocyte necrosis was observed. Furthermore, there were no significant signs of rejection in the heart. Because a remarkable difference of the survival times was observed between group B and group C on day 14, the histological comparison between group B and group C on day 14 was performed. Consequently, histological examination of grafts in group B showed end-stage rejection, and all the grafts had prominent muscle degeneration and severe lymphocyte infiltration in the perivascular area and interstitial space. However, the grafts in group C showed mild rejection with lymphocyte infiltration at the perivascular and interstitial spaces. Myocyte necrosis was not seen. These changes were located in the right ventricle.

The serum level of $GM_3$ administered was far below the physiological dose, and no direct toxicity to any tissue was observed. Diarrhea, abnormal behavior or death from an unknown cause was not observed in the rats during and after $GM_3$ administration.

## INDUSTRIAL APPLICABILITY

According to the present invention, there is provided an immunosuppressive agent which is advantageous, for example, in that (i) it can prevent rejection in organ transplantation, (ii) it permits local immunosuppression and hence reduction of systemic side effects observed in the case of conventional immunosuppressive agents, and (iii) it has neither systemic nor local toxicity.

## Claims

1. An immunosuppressive agent comprising N-acetylneuraminyl-galactosylceramide as an active ingredient.

2. An immunosuppressive agent according to Claim 1, which is intended for preventing immunological rejection in organ transplantation.

3. An immunosuppressive agent according to Claim 1, which uses N-acetylneuraminyl-galactosylceramide obtained from bovine brain.

4. An immunosuppression method comprising administration of an effective amount of N-acetylneuraminyl-galactosylceramide to any of warm-blooded animals including human beings.

5. An immunosuppression method according to Claim 4, which prevents immunological rejection in organ transplantation.

6. An immunosuppression method according to Claim 5, wherein the administration is local administration into a transplanted organ.

7. An immunosuppression method according to Claim 5, wherein N-acetylneuraminyl-galactosylceramide is administered at a dose of about 2 mg or more per 100 g (based on wet basis) of a transplanted organ.

8. Use of N-acetylneuraminyl-galactosylceramide for producing an immunosuppressive agent.

9. Use according to Claim 8, which is employment as an immunosuppressive agent for preventing immunological rejection in organ transplantation.

F I G. 1

GROUP C
(GROUP WHICH WAS
GIVEN GM₃ FOR 6
DAYS)

GROUP A
(CONTROL GROUP
WHICH WAS NOT
GIVEN GM₃)

GROUP B
(GROUP WHICH
WAS GIVEN GM₃
FOR 3 DAYS)

GRAFT SURVIVAL RATE (%)

POST-TRANSPLANT DAYS

EP 0 517 916 A1

# INTERNATIONAL SEARCH REPORT

International Application No PCT/JP91/00267

## I. CLASSIFICATION OF SUBJECT MATTER (if several classification symbols apply, indicate all) 6

According to International Patent Classification (IPC) or to both National Classification and IPC

Int. Cl$^5$   A61K31/70

## II. FIELDS SEARCHED

Minimum Documentation Searched 7

| Classification System | Classification Symbols |
| --- | --- |
| IPC | A61K31/70, 31/71, C07D15/04, 15/10 |

Documentation Searched other than Minimum Documentation
to the Extent that such Documents are Included in the Fields Searched 8

## III. DOCUMENTS CONSIDERED TO BE RELEVANT 9

| Category* | Citation of Document, 11 with indication, where appropriate, of the relevant passages 12 | Relevant to Claim No. 13 |
| --- | --- | --- |
| P | Chemical Abstracts Vol.113, No.25, page 36 (1990), 113:224285m | 1-3, 8, 9 |
| X | Journal of Immunology Vol.140, No.9, pages 3244 to 3248 (1988), It is described that GM3 exhibits immunosuppression. | 1-3, 8 |

* Special categories of cited documents: 10

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

## IV. CERTIFICATION

| Date of the Actual Completion of the International Search | Date of Mailing of this International Search Report |
| --- | --- |
| May 10, 1991 (10. 05. 91) | May 27, 1991 (27. 05. 91) |

| International Searching Authority | Signature of Authorized Officer |
| --- | --- |
| Japanese Patent Office | |

Form PCT/ISA/210 (second sheet) (January 1985)